# EUROPEAN PATENT SPECIFICATION

(11) **EP 3 874 518 B1**
(45) Date of publication and mention of the grant of the patent: **16.10.2024**
(21) Application number: 19784109.1
(22) Date of filing: 16.10.2019
(51) Int. Cl.: G16H 40/63, G16H 20/30, A61N 5/06

(54) **METHOD AND APPARATUS FOR PROVIDING USER FEEDBACK**
VERFAHREN UND VORRICHTUNG ZUR BEREITSTELLUNG EINER BENUTZERRÜCKMELDUNG
PROCÉDÉ ET APPAREIL POUR FOURNIR UN FEEDBACK UTILISATEUR

(30) Priority: 01.11.2018 EP 18203931
(43) Date of publication of application: 08.09.2021
(73) Proprietor: UNILEVER GLOBAL IP LIMITED, Wirral Merseyside CH62 4ZD (GB); Unilever IP Holdings B.V., 3013 AL Rotterdam (NL)
(72) Inventor: SAVILL, Derek, Guy, Wirral Merseyside CH63 3JW (GB); TRELOAR, Robert, Lindsay, Wirral Merseyside CH63 3JW (GB); ZILLMER, Ruediger, Wirral Merseyside CH63 3JW (GB)
(74) Representative: Tansley, Sally Elizabeth
(86) International application number: PCT/EP2019/078109
(87) International publication number: WO 2020/088938

(56) References cited:
- WO-A1-2017/157411
- WO-A1-2018/060232
- US-A1- 2012 029 417

## Description

This disclosure relates to methods and apparatus for assisting a user in a personal grooming activity.

WO 2018/060232 discloses an apparatus that has a control unit that is configured to determine a personal care activity performed by the user. The control unit identifies the regions of interest in the image of the user based on the determined personal care activity. The control unit acquires information associated with the determined personal care activity. The control unit modifies the image of the at user at the identified one or more regions of interest based on the acquired information to support the user in performing the personal care activity. The modified image is presented on a user interface ("smart mirror") and is not projected directly onto the user's face as in the present invention.

The prior art teaches a number of systems for providing feedback on toothbrushing activity, including providing a user with an indication as to whether particular regions of the mouth have been adequately or sufficiently brushed. Various systems have been proposed for monitoring toothbrushing activity including those using sensors such as accelerometers located on or in the toothbrush, as well as those that use a camera and image tracking systems to observe the brushing position and brushing angle adopted by the user.

Various display systems are proposed for providing the user with visual feedback as to the quality and extent of tooth brushing. Various problems can arise with displaying such visual feedback to the user. A display on the brush or other device may not generally be in the user's line of sight during brushing and therefore could be sub-optimal for providing continuous, real-time feedback because the user may need to stop brushing activity or look away from a mirror to observe the display.

Some mirror display systems, which may be referred to as 'smart mirrors', have been proposed that not only reflect a user's image in a conventional way, but also incorporate a display screen under the mirror surface for displaying images thereon. A camera module and processor are used to locate a user's eyes and determine a line of sight of the user (i.e. the gaze direction). The mirror display system aims to create an image or other digital content on the display screen in a position which is coincident with the line of sight of the user to a virtual position of a reflected object behind the plane of the mirror at which the user is looking. Problems can arise with the accuracy of positioning of the displayed image in x-y space (horizontal and vertical positioning) such that the image remains in the line of the user's sight or gaze direction, and problems can also arise because the displayed image or other digital content may not be in focus for the user, given that the user's gaze may be directed at a virtual image in z-space (depth positioning) well behind the plane of the mirror.

In one aspect, the invention seeks to overcome such problems. In another aspect, the invention seeks to provide an alternative method and apparatus for providing feedback to a user, for example, while carrying out personal grooming activities, such as tooth brushing.

According to one aspect, the invention provides a method of providing visual feedback to a user as described in claim 1.

The personal grooming activity comprises tooth brushing and the at least one item of feedback may comprise indicating sufficient or insufficient level of brushing in plural brushing regions of the mouth. The visual projection of feedback information may comprise giving a visual indication of a level of brushing, on the user's face in a position corresponding to the brushing region.

Monitoring the personal grooming activity may comprise tracking the position, motion and/or orientation of a toothbrush relative to the user's mouth. Tracking the position, motion and/or orientation of the toothbrush may comprise detecting markers or patterns on the toothbrush. Tracking the position, motion and/or orientation of the toothbrush may comprise receiving sensed data from an accelerometer or other movement sensor embedded on or in the toothbrush. The feedback may comprise projection of a colour coded block, a line or other shape of light to a region of the mouth to which brushing is being or has been applied, indicative of a level of adequacy of brushing applied to the region. The brushing feedback may divide the teeth surfaces into a number of regions including upper teeth and lower teeth, and optionally anterior, left and right teeth rows, and the colour-coded block, the line, or other shape of light may be projected for respective regions onto left cheek, right cheek and mouth front, above or below the lip line. The projected feedback information may comprise multilevel information corresponding to different parameters of the tooth brushing activity which are colour-coded, temporally separated, spatially separated or distinguished by projected words or numbers or symbols. The different parameters may comprise two or more of duration of brushing, position of brushing, correctness of brushing technique, pressure applied during brushing.

The method includes: determining a position of the user's eyes; and excluding or inhibiting projected light of said projected information from reaching an area corresponding to the determined position of the user's eyes. The method may further include performing the preceding steps continuously in real time so that the relevant location maintains a spatial correlation with a tracked position of the target region of the user's body.

The personal grooming activity comprises tooth brushing. The at least one item of feedback comprises indicating sufficient or insufficient level of brushing in plural brushing regions of the mouth. The visual projection of feedback information gives a visual indication of a level of brushing, on the user's face in a position corresponding to the brushing region.

The monitoring of a personal grooming activity may comprise: directing diagnostic electromagnetic radiation at the user's body and receiving a response signal therefrom, correlating the response signal with the tracked position of the user's body, and determining an item of feedback based on the response signal. The monitoring of a personal grooming activity may comprise using image analysis to determine a pattern of movement of the user and / or a toothbrush in performing the personal grooming activity.

Steps as described above may be performed by a smartphone or by a smartphone and a projector in wireless communication with one another.

The target region may be at least part of the head of the user.

According to another aspect, the invention provides an apparatus for providing visual feedback to a user during personal grooming activities as described in claim 11 .

The activity monitoring module may be configured to track the position, motion and/or orientation of a toothbrush relative to the user's mouth. The activity tracking module may be configured to detect markers or patterns on the toothbrush. The activity tracking module may be configured to receive sensed data from an accelerometer or other movement sensor embedded on or in the toothbrush. The projection control module may be configured to provide feedback comprising projection data for a colour coded block, a line or other shape of light to be projected to a region of the mouth to which brushing is being or has been applied, indicative of a level of adequacy of brushing applied to the region. The projection control module may be configured to provide brushing feedback which divides the teeth surfaces into a number of regions including upper teeth and lower teeth, and optionally anterior, left and right teeth rows, and the colour-coded block, the line, or other shape of light is to be projected for respective regions onto left cheek, right cheek and mouth front, above or below the lip line. The feedback module may be configured to provide feedback information comprising multilevel information corresponding to different parameters of the tooth brushing activity which are colour-coded, temporally separated, spatially separated or distinguished by projected words or numbers or symbols. The different parameters may comprise two or more of duration of brushing, position of brushing, correctness of brushing technique, pressure applied during brushing.

The apparatus includes a sensor for determining a position of the user's eyes and a blocking module configured to inhibit projection of light by the projector from reaching an area corresponding to the determined position of the user's eyes. The activity module may include a toothbrush motion sensing device for determining a toothbrushing pattern of the user. The feedback module may be configured to determine one or more portions of the face corresponding to regions of the toothbrushing pattern for projecting feedback.

The apparatus may further include a diagnostic device configured to direct diagnostic electromagnetic radiation at the user's body and receive a response signal indicative of a condition of at least a part of the user's body, and wherein the feedback module is configured to determine at least one item of feedback based on the response signal. The activity monitoring module may be configured to monitor the personal grooming activity using image signals from the camera. The camera, the activity monitoring module and/or the feedback module may be disposed within a smartphone. The apparatus may be integrated into a mirror. The apparatus may further include a projector configured to receive the output of the projection control module and project the information onto a user's body at the relevant location.

Embodiments of the present invention will now be described by way of example and with reference to the accompanying drawings in which:
Figure 1 shows a schematic perspective view of an apparatus for providing visual feedback to a user during a personal grooming activity;
Figure 2 shows a schematic functional block diagram of components of the apparatus of figure 1;
Figure 3 shows a flow chart of the functions that may be performed by the apparatus of figure 1.

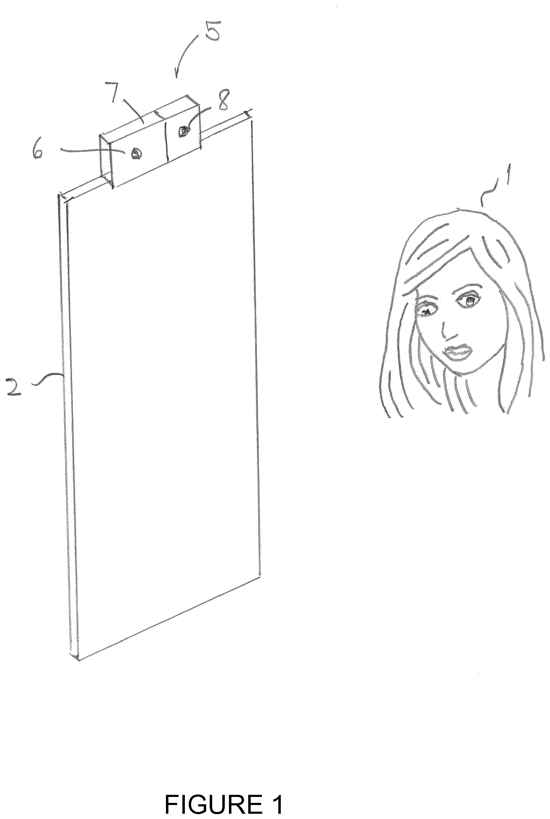

A visual feedback system is shown in figure 1. A user 1 is positioned in front of a mirror 2 in order to undertake some personal grooming activity. The expression 'personal grooming activity' is intended to encompass toothcare activities, skin care activities, hair care activities, and the application of cosmetic, beauty or medical products. Examples of such personal grooming activities may include brushing the teeth, flossing the teeth or cleaning interdental spaces, hair brushing, hair styling or application of hair product, application of skin treatments or make-up.

The visual feedback system 5 comprises: a camera 6 which is configured to determine / track the position of at least a portion of the user's body, e.g. the head; a feedback device 7 configured to determine at least one item of feedback relevant to the user's personal grooming activity and to determine a relevant location on the user's body onto which to provide a visual projection of information indicative of the item(s) of feedback; and a projector 8 configured to project the information onto the user's body at the relevant location.

In many examples, the personal grooming activity is being carried out on or in relation to the user's head and the projector 8 is configured to project the information onto the head. This is particularly applicable to giving feedback on tooth brushing activities and hair care activities including hair styling and application of facial care products.

The camera 6 and feedback device 7 and projector 8 could be integrated into a single, standalone housing, or could be integrated into a frame of the mirror 2. In another possible arrangement, the camera 6 and feedback device 7 could be implemented in a smartphone which can be positioned on a shelf, ledge or other support structure adjacent to or forming part of the mirror 2. It is also envisaged that the projector 8 could be separately provided and communicate with, for example, a smartphone implementation of the camera and feedback device, using any suitable communication channel such as wireless communication using Bluetooth, WiFi or NFC channels. In another example, a smartphone may incorporate a microprojector system capable of projecting the required feedback information onto the user's body. The expression 'camera' as used herein is intended to encompass any imaging device capable of providing information indicative of the spatial location of relevant parts of the user's body, suitable for tracking the variable positions thereof.

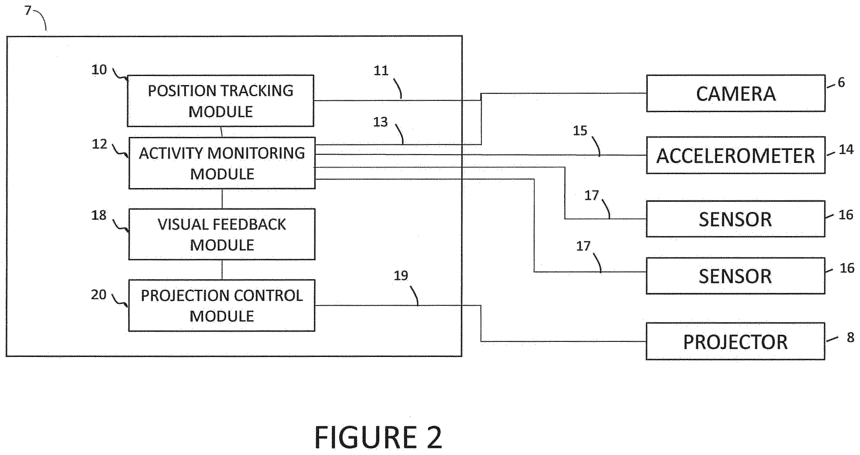

Figure 2 shows a schematic functional block diagram of the various functions which may be implemented in the feedback device 7.

A position tracking module 10 is configured to receive image data from the camera 6 or other imaging / tracking device over a communication channel 11 and to determine a position of a target region of a user's body. The target region may comprise the region of a user's body to which the personal grooming activity is being applied. Thus, for toothcare activities, the target region comprises the mouth area.

An activity monitoring module 12 is configured to monitor the personal grooming activity in the target region. In some examples, the monitoring of personal grooming activity may be performed by analysis of images delivered from the camera 6 over communication channel 13. The analysis of images may include monitoring a pattern of movement of the user or an appliance held by the user. For example, tracking of the position, motion and/or orientation of a toothbrush relative to the user's mouth by detection of specific markers or patterns on the toothbrush is possible, relative to the position of the user's head.

In other examples, monitoring of personal grooming activity may be performed by use of data received from other types of sensor, such as an accelerometer or other movement sensor 14 embedded on or in a toothbrush. The accelerometer or other movement sensor 14 may provide data to the activity monitor by any suitable communication channel 15 such a wireless channel. Various systems are known in the art for tracking toothbrush motion on different surfaces of the teeth and in different positions in the mouth using accelerometer data and computing a degree of brushing thoroughness in different locations of the mouth therefrom. In this way, feedback on the progress of toothbrushing may be given.

In another example, the sensors 16 may include an active sensor for directing diagnostic electromagnetic radiation at the target region of the user's body to gather electromagnetic response signals that may be indicative of properties of the user's body,. These response signals would be correlated with the tracked position of the user's body to enable the provision of feedback based thereon. In one example, the sensor 16 may be configured to determine a depth of product applied, a colour of product applied or some other physical parameter capable of indicating a quality or quantity of the product applied. In this way, feedback may be given regarding depth or consistency or uniformity of the application of a product being applied. The sensors 16 may comprise other types of sensor such as passive sensors for gathering electromagnetic response signals from ambient light reflected from the user's body. Other types of sensor 16 could include hyperspectral imaging sensors for mapping properties of the target region of the user's body.

A feedback module 18 is configured to determine, from the output of the activity monitoring module 12, one or more items of feedback that are appropriate to deliver to the user, and to determine an appropriate position on the user's body to which each item of feedback should be delivered. In the example of providing toothcare feedback, the feedback may comprise, for example delivery of a colour coded block, line or other shape of light to each region of the mouth to which brushing is being or has been applied. Typical brushing feedback may divide the teeth surfaces into a number of regions, e.g. upper teeth and lower teeth, where each may be subdivided into anterior, left and right teeth rows, and these rows may be further subdivided into lingual (tongue-side), buccal (cheek-side) and occlusal surfaces. Some or all of these specific regions and/or surfaces can be indicated by projection of a coloured light or light pattern respectively onto left cheek, right cheek and mouth front, above or below the lip line, for example. An area of green projected light may indicate sufficient or adequate brushing in the selected region; an area of red projected light may indicate no brushing completed in the selected region; and an area of amber projected light may indicate insufficient brushing completed in the selected region. Other indicators of a level of brushing may be envisaged. These indicators may be projected in series to indicate the next region of the teeth to be brushed and display colour change as the task is successively completed in each region. Feedback indicators could also or alternatively include, for example, information as to whether toothbrushing was being performed too vigorously such as applying too much pressure, or missing certain parts of the teeth surfaces. More generally, projected feedback information may comprise multilevel information corresponding to different parameters of the personal grooming activity which can be colour-coded, temporally separated, spatially separated or distinguished in other ways such as by projected words or numbers or symbols. In this way, multiple items of feedback can be provided simultaneously or near-simultaneously relating to the monitored grooming activity, each corresponding to a different monitored parameter of the grooming activity, such as duration of brushing, position of brushing, correctness of brushing technique, etc.

Similarly, in the example of application of products, projected colour or other visual content may be used to indicate regions where sufficient, or insufficient, quantity of product has been applied.

The feedback device 7 further includes a projection control module 20 which is configured to control a projector 8, over communication line 19, to direct the projection of feedback items to the relevant location on the user's body as discussed above.

The feedback device 7 is preferably configured to track the position of the target region of the user's body continuously in real time and to continuously update in real time the position of the relevant location for projection of the feedback based on the position of the user's body. In this way, the relevant location for projection remains spatially correlated with the tracked position of the target region of the user's body. In this respect, the feedback device 7 may implement a dynamic projection mapping system adapted to ensure the feedback items are projected to the relevant location during movement of the user's body and therefore during movement of the relevant location on the user's body. In many cases, the target region where the personal grooming activity is taking place will be the same as, within, or close to, the relevant location where the feedback information is to be projected. This can assist in ensuring that the direction of the user's gaze, and the focus of the user's eyes, are optimal for monitoring the personal grooming activity and the feedback information given. This offers a considerable advantage over existing feedback systems where a separate display is provided away from the user's normal sight line or where the virtual display is not in the plane of the user's eye focus.

The feedback device 7 may also be configured such that the position tracking module 10 determines a current position of the user's eyes, preferably updated continuously or near-continuously, and ensures that the projector 8 is prevented from projecting light into the user's eyes. This may be implemented by determining a mobile exclusion area or projection mask area corresponding to the determined eye position (which may include a margin or penumbra of overlap / protection) into which the projection control module 20 is prevented from instructing the projector 8, while simultaneously enabling continued projection of feedback information outside the projection mask area. In this way, the user is protected from being dazzled or otherwise distracted during a personal grooming activity, even while performing such grooming activities on an area of the face close to the eyes.

Identification of a target region of a user's body may be effected either automatically, or by user input. For example, the feedback device may be configured to determine the user's line of sight and deduce the portion of the body to which attention is being directed, and/or detect a pattern of movement of the user (e.g. arms or hands) in relation thereto, thereby enabling the device to deduce a specific personal grooming activity using a template matching algorithm. Feedback can then be provided based on the identified personal grooming activity. Alternatively, the target region may be identified by activation of, for example a grooming device such as a toothbrush in communication with the feedback device 7. Detection of toothbrush activity can then be used to automatically establish the target region as being the mouth area. In another example, the user may be required to select a personal grooming activity from a menu on an input device, e.g. the user's smartphone, which will then determine the target area for tracking.

The projected visual feedback can readily be viewed by a user looking at his or her body through the mirror. Because the user is focused on the personal grooming activity by looking through the mirror at the target region of the body (e.g. head), the feedback information will automatically be in line with the user's gaze direction and also in focus for the user's eyes because it normally lies in or close to the same focal plane as the target region. The dynamic masking function which tracks the user's eyes and is applied to the light projection ensures that the user's eyes are always shielded from a projected feedback image.

A further advantage over a 'smart mirror' solution as discussed above is that the described system requires no display panel integrated with the mirror, since the feedback display is implemented by projection of light onto the user. This makes the system backwardly compatible with a user's ordinary bathroom mirror, and no special mirror hardware is required. The system can be implemented by way of a low cost pico-projector and camera system alongside an existing mirror or mounted nearby, and the (or at least most of the) required hardware may be incorporated into user's smartphone.

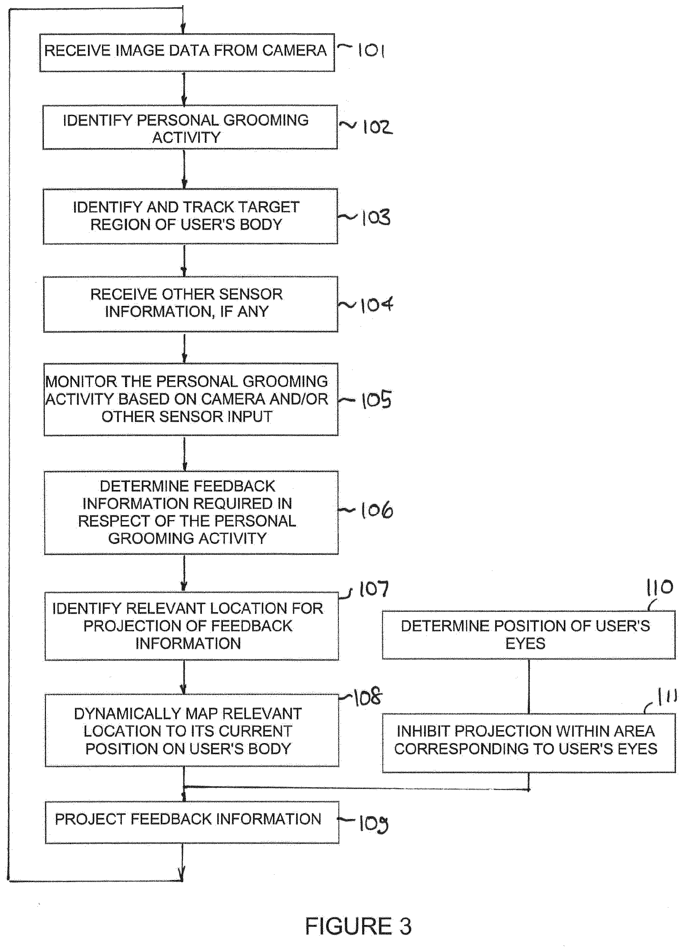

Figure 3 shows a flow chart of the functions that may be performed by the feedback device 7. Image data is received from the camera for providing to a position tracking module 10 (box 101). A personal grooming activity is identified (box 102), which may be performed by analysis of the image data received from the camera 6, or by analysis of data received from one or more other sensors 14, 16, or by specific sensors 14, 16 provided within a personal grooming appliance such as an electronic toothbrush, or by receiving user input indicating a user's intended activity. The target region of the user's body relevant to the identified personal grooming activity is identified (box 103). It will be recognised that the functions of identifying the personal grooming activity and identifying the target region may be performed contemporaneously, or in reverse order, e.g. where the personal grooming activity is deduced from the region of the user's body in which movement is being tracked. Other sensor information may be received by an activity monitoring module 12 (box 104) such as from an accelerometer 14 or other motion sensor, from an infrared sensor 16 or other thermal imaging device, from an electromagnetic radiation sensor 16 (passive or active), or from a hyperspectral imaging sensor 16, for example. The various inputs as described are received and the progress of a personal grooming activity is monitored (box 105). The activity monitoring module 12 is operative to determine one or more items of visual feedback that can be given to the user based on the progress of the personal grooming activity (box 106). This feedback may include, for example, visual indications of regions of a user's mouth for which toothbrushing activity has been sufficiently, or insufficiently completed. A relevant location for the projection of the visual feedback information is then determined (box 107), e.g. positions on the user's face (e.g. cheeks or above / below the lips for the toothbrush example above). The current position of the relevant location on the user's body is then determined by dynamic mapping based on the received images from the camera 6 (box 108) and the visual feedback information is projected onto the relevant location (box 109). The process is executed continuously or near-continuously, as indicated by the loop back to box 101, during the personal grooming activity. Thus, the feedback device can operate a self-correcting loop of detecting progress of the personal grooming activity, determining the position of the location for projection of feedback and updating the projection feedback to be delivered. In parallel with the above, the system may be configured to determine, in real time, the position of the user's eyes (box 110) and use this information to inhibit any projection of visual feedback within the area corresponding to the user's eyes (box 111), so as to prevent the user being dazzled or otherwise distracted during the personal grooming activity. The functions illustrated by box 110 and box 111 also may be on a continuous or near-continuous execution loop.

Throughout the present specification, the expression 'module' is intended to encompass a functional system which may comprise computer code being executed on a generic or a custom processor, or a hardware machine implementation of the function, e.g. on an application-specific integrated circuit.

The specification has described various types of visual feedback based on monitoring of a personal grooming activity. The monitoring of the grooming activity, to serve as the basis for providing feedback on the grooming activity, can be performed in a wide range of ways such as image analysis and position tracking using accelerometers or movement sensors, including magnetic field-based sensing systems such as described in WO 2018/065373, and learning-based systems which derive a user-specific statistical model that maps positional information onto movement information so that the model can then be used to provide positional information, e.g. of a toothbrush, from sensed movement information, as described in WO 2018/065372. Other examples are within the reach of the skilled person, and combinations of such methods may be used.

The expressions 'track' and 'tracking' as used herein are intended to encompass situations in which the user may be moving, e.g. in front of a mirror, such that a new position of the user is being regularly determined, but may also encompass situations where the user is relatively static in front of the mirror such that a same position of the user is being regularly affirmed.

Other embodiments are intentionally within the scope of the accompanying claims.

## Claims

1. A method for providing visual feedback to a user during personal grooming activities, comprising:
determining a position of a target region of the user's body;
monitoring a personal grooming activity of the user and determining at least one item of feedback relevant to the user's personal grooming activity;
determining a relevant location on the user's body for visual projection of information indicative of the item of feedback;
projecting said information onto the user's body at the relevant location,
wherein the personal grooming activity comprises tooth brushing and the at least one item of feedback comprises indicating sufficient or insufficient level of brushing in plural brushing regions of the mouth, and in which the visual projection of feedback information comprises giving a visual indication of a level of brushing, on the user's face in a position corresponding to the brushing region,
wherein the method further comprises:
determining a position of the user's eyes; and
excluding or inhibiting projected light of said projected information from reaching an area corresponding to the determined position of the user's eyes.

2. The method as claimed in claim 1 in which monitoring the personal grooming activity comprises tracking of the position, motion and/or orientation of a toothbrush relative to the user's mouth.

3. The method as claimed in claim 2 in which tracking of the position, motion and/or orientation of the toothbrush comprises detecting markers or patterns on the toothbrush.

4. The method as claimed in claim 2 in which tracking of the position, motion and/or orientation of the toothbrush comprises receiving sensed data from an accelerometer or other movement sensor embedded on or in the toothbrush.

5. The method as claimed in claim 1 in which the feedback comprises projection of a colour coded block, a line or other shape of light to a region of the mouth to which brushing is being or has been applied, indicative of a level of adequacy of brushing applied to the region.

6. The method as claimed in claim 5 in which the brushing feedback divides the teeth surfaces into a number of regions including upper teeth and lower teeth, and optionally anterior, left and right teeth rows, and the colour-coded block, the line, or other shape of light is projected for respective regions onto left cheek, right cheek and mouth front, above or below the lip line.

7. The method as claimed in claim 1 in which the projected feedback information comprises multilevel information corresponding to different parameters of the tooth brushing activity which are colour-coded, temporally separated, spatially separated or distinguished by projected words or numbers or symbols.

8. The method as claimed in claim 7 in which different parameters comprise two or more of duration of brushing, position of brushing, correctness of brushing technique, pressure applied during brushing.

9. The method as claimed in claim 1 in which excluding or inhibiting projected light of said projected information from reaching an area corresponding to the determined position of the user's eyes comprises determining an exclusion area or projection mask area corresponding to the determined eye position into which a projection control module is prevented from instructing a projector, while simultaneously enabling continued projection of feedback information outside the projection mask area.

10. The method as claimed in claim 1 further including performing the steps of claim 1 or claim 9 continuously in real time so that the relevant location maintains a spatial correlation with a tracked position of the target region of the user's body.

11. Apparatus for providing visual feedback to a user during tooth brushing, comprising:
a camera configured to determine a position of a target region of the user's body;
an activity monitoring module configured to monitor a tooth brushing activity of the user;
a feedback module configured to determine at least one item of feedback relevant to the user's tooth brushing activity and to determine a relevant location on the user's body for visual projection of information indicative of the item of feedback, the at least one item of feedback comprising indicating sufficient or insufficient level of brushing in plural brushing regions of the mouth;
a projection control module configured to provide output for a projector to visually project the information onto the user's body at the relevant location, the feedback information for visual projection comprising a visual indication of a level of brushing, for projection on the user's face in a position corresponding to the brushing region;
a sensor for determining a position of the user's eyes and
a blocking module configured to inhibit projection of light by the projector from reaching an area corresponding to the determined position of the user's eyes.

12. The apparatus as claimed in claim 11 in which the activity monitoring module is configured to track the position, motion and/or orientation of a toothbrush relative to the user's mouth.

13. The apparatus as claimed in claim 12 in which the activity tracking module is configured to detect markers or patterns on the toothbrush.

14. The apparatus as claimed in claim 12 in which the activity tracking module is configured to receive sensed data from an accelerometer or other movement sensor embedded on or in a toothbrush.

15. The apparatus as claimed in claim 14 in which the projection control module is configured to provide feedback comprising projection data for a colour coded block, a line or other shape of light to be projected to a region of the mouth to which brushing is being or has been applied, indicative of a level of adequacy of brushing applied to the region.

## Patentansprüche

1. Verfahren zum Bereitstellen einer optischen Rückmeldung für einen Benutzer während persönlicher Pflegeaktivitäten, wobei das Verfahren die folgenden Schritte umfasst:
Ermitteln einer Position eines Zielbereichs des Körpers des Benutzers;
Überwachen einer persönlichen Pflegeaktivität des Benutzers und Ermitteln wenigstens eines Rückmeldungselements, das für die persönliche Pflegeaktivität des Benutzers relevant ist,
Ermitteln einer relevanten Position am Körper des Benutzers zur optischen Projektion von Informationen, die das Rückmeldungselement anzeigen,
Projizieren der Informationen auf den Körper des Benutzers an der relevanten Position,
wobei die persönliche Pflegeaktivität Zähneputzen umfasst und das wenigstens eine Rückmeldungselement das Anzeigen eines ausreichenden oder nicht ausreichenden Putzgrads in mehreren Putzbereichen des Munds umfasst und wobei die optische Projektion der Rückmeldungsinformationen das Bereitstellen einer optischen Anzeige eines Putzgrads auf dem Gesicht des Benutzers an einer Position entsprechend dem Putzbereich umfasst,
wobei das Verfahren ferner die folgenden Schritte umfasst:
Ermitteln einer Position der Augen des Benutzers; und
Ausschließen oder Verhindern, dass projiziertes Licht der projizierten Informationen einen Bereich erreicht, der der ermittelten Position der Augen des Benutzers entspricht.

2. Verfahren nach Anspruch 1, wobei das Überwachen der persönlichen Pflegeaktivität das Nachverfolgen der Position, der Bewegung und/oder der Orientierung einer Zahnbürste relativ zum Mund des Benutzers umfasst.

3. Verfahren nach Anspruch 2, wobei das Nachverfolgen der Position, der Bewegung und/oder der Orientierung der Zahnbürste das Detektieren von Markierungen oder Mustern an der Zahnbürste umfasst.

4. Verfahren nach Anspruch 2, wobei das Nachverfolgen der Position, der Bewegung und/oder der Orientierung der Zahnbürste das Erhalten von Messdaten von einem Beschleunigungsmessgerät oder einem anderen Bewegungssensor, der an oder in der Zahnbürste eingebettet ist, umfasst.

5. Verfahren nach Anspruch 1, wobei die Rückmeldung die Projektion eines farbcodierten Blocks, einer Linie oder einer anderen Form von Licht auf einen Bereich des Munds, bei dem das Putzen gerade ausgeführt wird oder ausgeführt worden ist, umfasst, die einen Grad der Angemessenheit des Putzens anzeigt, das in dem Bereich ausgeführt worden ist.

6. Verfahren nach Anspruch 5, wobei die Putzrückmeldung die Zahnflächen in eine Anzahl von Bereichen unterteilt, die obere Zähne und untere Zähne und optional Frontzähne, linke und rechte Zahnreihen umfassen, und wobei der farbcodierte Block, die Linie oder die andere Form von Licht für jeweilige Bereiche auf die linke Wange, die rechte Wange und die Mundvorderseite über oder unter der Lippenlinie projiziert wird.

7. Verfahren nach Anspruch 1, wobei die projizierten Rückmeldungsinformationen mehrstufige Informationen umfassen, die unterschiedlichen Parametern der Zahnputzaktivität entsprechen, die farbcodiert, zeitlich getrennt, räumlich getrennt oder durch projizierte Wörter oder Ziffern oder Symbole unterschieden werden.

8. Verfahren nach Anspruch 7, wobei die unterschiedlichen Parameter zwei oder mehr aus der Putzdauer, der Putzposition, der Korrektheit der Putztechnik und dem Druck, der während des Putzens ausgeübt wird, umfassen.

9. Verfahren nach Anspruch 1, wobei das Ausschließen oder Verhindern, dass projiziertes Licht der projizierten Informationen einen Bereich erreicht, der der ermittelten Position der Augen des Benutzers entspricht, das Ermitteln eines Ausschlussbereichs oder eines Projektionsmaskenbereichs entsprechend der ermittelten Augenposition umfasst, indem verhindert wird, dass ein Projektionssteuermodul einen Projektor anweist, während gleichzeitig eine fortgesetzte Projektion von Rückmeldungsinformationen außerhalb des Projektionsmaskenbereichs möglich ist.

10. Verfahren nach Anspruch 1, das ferner das fortgesetzte Ausführen der Schritte von Anspruch 1 oder Anspruch 9 in Echtzeit umfasst, so dass die relevante Position eine räumliche Korrelation mit einer nachverfolgten Position des Zielbereichs des Körpers des Benutzers beibehält.

11. Vorrichtung zum Bereitstellen einer optischen Rückmeldung für einen Benutzer während des Zähneputzens, wobei die Vorrichtung Folgendes umfasst:
eine Kamera, die konfiguriert ist, eine Position eines Zielbereichs des Körpers des Benutzers zu ermitteln,
ein Aktivitätsüberwachungsmodul, das konfiguriert ist, eine Zahnputzaktivität des Benutzers zu überwachen;
ein Rückmeldungsmodul, das konfiguriert ist, wenigstens ein Rückmeldungselement zu ermitteln, das für die Zahnputzaktivität des Benutzers relevant ist, und eine relevante Position am Körper des Benutzers für eine optische Projektion von Informationen zu ermitteln, die das Rückmeldungselement anzeigen, wobei das wenigstens eine Rückmeldungselement das Anzeigen eines ausreichenden oder nicht ausreichenden Putzgrads in mehreren Putzbereichen des Munds umfasst,
ein Projektionssteuermodul, das konfiguriert ist, ein Ausgangssignal für einen Projektor bereitzustellen, um die Informationen optisch auf den Körper des Benutzers an der relevanten Position zu projizieren, wobei die Rückmeldungsinformationen für eine optische Projektion eine optische Anzeige eines Putzgrads zur Projektion auf das Gesicht des Benutzers an einer Position, die dem Putzbereich entspricht, umfassen;
einen Sensor zum Ermitteln einer Position der Augen des Benutzers, und
ein Blockiermodul, das konfiguriert ist, zu verhindern, dass eine Projektion von Licht durch den Projektor einen Bereich erreicht, der der ermittelten Position der Augen des Benutzers entspricht.

12. Vorrichtung nach Anspruch 11, wobei das Aktivitätsüberwachungsmodul konfiguriert ist, die Position, die Bewegung und/oder die Orientierung einer Zahnbürste relativ zum Mund des Benutzers nachzuverfolgen.

13. Vorrichtung nach Anspruch 12, wobei das Modul zum Nachverfolgen von Aktivitäten konfiguriert ist, Markierungen oder Muster an der Zahnbürste zu detektieren.

14. Vorrichtung nach Anspruch 12, wobei das Modul zum Nachverfolgen von Aktivitäten konfiguriert ist, Messdaten von einem Beschleunigungsmessgerät oder einem anderen Bewegungssensor, der an oder in einer Zahnbürste eingebettet ist, zu erhalten.

15. Vorrichtung nach Anspruch 14, wobei das Modul zum Steuern der Projektion konfiguriert ist, eine Rückmeldung bereitzustellen, die Projektionsdaten für einen farbcodierten Block, eine Linie oder eine andere Form von Licht umfasst, die auf einen Bereich des Munds, bei dem das Putzen ausgeführt wird oder ausgeführt worden ist, projiziert werden, die einen Grad der Angemessenheit des Putzens, das in dem Bereich ausgeführt worden ist, anzeigen.

## Revendications

1. Procédé pour la fourniture d'un retour visuel à un utilisateur pendant des activités d'hygiène personnelle, comprenant :
la détermination d'une position d'une région cible du corps de l'utilisateur ;
la surveillance d'une activité d'hygiène personnelle de l'utilisateur et la détermination d'au moins un élément de retour pertinent pour l'activité d'hygiène personnelle de l'utilisateur ;
la détermination d'un emplacement pertinent sur le corps de l'utilisateur pour une projection visuelle d'informations indicatives de l'élément de retour ;
la projection desdites informations sur le corps de l'utilisateur à l'emplacement pertinent,
dans lequel l'activité d'hygiène personnelle comprend un brossage des dents et l'au moins un élément de retour comprend l'indication d'un niveau de brossage suffisant ou insuffisant dans plusieurs régions de brossage de la bouche, et dans lequel la projection visuelle d'informations de retour comprend le fait de donner une indication visuelle d'un niveau de brossage, sur le visage de l'utilisateur à une position correspondant à la région de brossage,
dans lequel le procédé comprend en outre :
la détermination d'une position des yeux de l'utilisateur ; et
le fait d'exempter ou d'empêcher une lumière projetée desdites informations projetées d'atteindre une zone correspondant à la position déterminée des yeux de l'utilisateur.

2. Procédé selon la revendication 1 dans lequel la surveillance de l'activité d'hygiène personnelle comprend le suivi de la position, du mouvement et/ou de l'orientation d'une brosse à dents par rapport à la bouche de l'utilisateur.

3. Procédé selon la revendication 2 dans lequel le suivi de la position, du mouvement et/ou de l'orientation de la brosse à dents comprend la détection de repères ou de motifs sur la brosse à dents.

4. Procédé selon la revendication 2 dans lequel le suivi de la position, du mouvement et/ou de l'orientation de la brosse à dents comprend la réception de données détectées provenant d'un accéléromètre ou d'un autre capteur de mouvement incorporé sur ou dans la brosse à dents.

5. Procédé selon la revendication 1 dans lequel le retour comprend la projection d'un bloc codé par couleur, d'une ligne ou d'une autre forme de lumière sur une région de la bouche sur laquelle le brossage est en cours d'application ou a été appliqué, indiquant un niveau d'adéquation de brossage appliqué sur la région.

6. Procédé selon la revendication 5 dans lequel le retour de brossage divise les surfaces des dents en un nombre de régions incluant des dents du haut et des dents du bas, et facultativement des rangées de dents antérieure, de gauche et de droite, et le bloc codé par couleur, la ligne ou l'autre forme de lumière est projeté(e) pour des régions respectives sur la joue gauche, la joue droite et le devant de la bouche, au-dessus ou au-dessous du contour des lèvres.

7. Procédé selon la revendication 1 dans lequel les informations de retour projetées comprennent des informations à niveaux multiples correspondant à différents paramètres de l'activité de brossage des dents qui sont codées par couleur, temporellement séparées, spatialement séparées ou se distinguent par des mots ou des nombres ou des symboles projetés.

8. Procédé selon la revendication 7 dans lequel différents paramètres comprennent deux éléments ou plus parmi la durée du brossage, la position du brossage, la justesse de la technique de brossage, la pression appliquée pendant le brossage.

9. Procédé selon la revendication 1 dans lequel le fait d'exempter ou d'empêcher une lumière projetée desdites informations projetées d'atteindre une zone correspondant à la position déterminée des yeux de l'utilisateur comprend la détermination d'une zone d'exemption ou zone de masque de projection correspondant à la position des yeux déterminée dans laquelle un module de commande de projection est empêché de donner des instructions à un projecteur, tout en permettant simultanément une projection continue d'informations de retour à l'extérieur de la zone de masque de projection.

10. Procédé selon la revendication 1 incluant en outre la réalisation des étapes de la revendication 1 ou de la revendication 9 en continu en temps réel de sorte que l'emplacement pertinent conserve une corrélation spatiale avec une position suivie de la région cible du corps de l'utilisateur.

11. Appareil pour la fourniture d'un retour visuel à un utilisateur pendant un brossage des dents, comprenant :
un appareil de prise de vues configuré pour déterminer une position d'une région cible du corps de l'utilisateur ;
un module de surveillance d'activité configuré pour surveiller une activité de brossage des dents de l'utilisateur ;
un module de retour configuré pour déterminer au moins un élément de retour pertinent pour l'activité de brossage des dents de l'utilisateur et pour déterminer un emplacement pertinent sur le corps de l'utilisateur pour une projection visuelle d'informations indicatives de l'élément de retour, l'au moins un élément de retour comprenant l'indication d'un niveau de brossage suffisant ou insuffisant dans plusieurs régions de brossage de la bouche ;
un module de commande de projection configuré pour fournir une sortie pour qu'un projecteur projette visuellement les informations sur le corps de l'utilisateur à l'emplacement pertinent, les informations de retour pour une projection visuelle comprenant une indication visuelle d'un niveau de brossage, pour une projection sur le visage de l'utilisateur à une position correspondant à la région de brossage ;
un capteur pour la détermination d'une position des yeux de l'utilisateur et
un module de blocage configuré pour empêcher une projection de lumière par le projecteur d'atteindre une zone correspondant à la position déterminée des yeux de l'utilisateur.

12. Appareil selon la revendication 11 dans lequel le module de surveillance d'activité est configuré pour suivre la position, le mouvement et/ou l'orientation d'une brosse à dents par rapport à la bouche de l'utilisateur.

13. Appareil selon la revendication 12 dans lequel le module de suivi d'activité est configuré pour détecter des repères ou des motifs sur la brosse à dents.

14. Appareil selon la revendication 12 dans lequel le module de suivi d'activité est configuré pour recevoir des données détectées provenant d'un accéléromètre ou d'un autre capteur de mouvement incorporé sur ou dans une brosse à dents.

15. Appareil selon la revendication 14 dans lequel le module de commande de projection est configuré pour fournir un retour comprenant des données de projection pour un bloc codé par couleur, une ligne ou une autre forme de lumière devant être projeté(e) sur une région de la bouche sur laquelle le brossage est en cours d'application ou a été appliqué, indiquant un niveau d'adéquation de brossage appliqué sur la région.
